# EUROPEAN PATENT SPECIFICATION

(11) **EP 3 897 731 B1**
(45) Date of publication and mention of the grant of the patent: **27.08.2025**
(21) Application number: 19839302.7
(22) Date of filing: 17.12.2019
(51) Int. Cl.: A61K 47/38, A61K 9/20, A61K 9/48, A61K 31/167, A61K 9/00

(54) **A SUSTAINED RELEASE COMPOSITION COMPRISING AN ETHYLCELLULOSE**
ZUSAMMENSETZUNG MIT EINER ETHYLCELLULOSE MIT VERZÖGERTER FREISETZUNG
COMPOSITION À LIBÉRATION PROLONGÉE COMPRENANT DE L'ÉTHYLCELLULOSE

(30) Priority: 18.12.2018 EP 18213448
(43) Date of publication of application: 27.10.2021
(73) Proprietor: DDP Specialty Electronic Materials US, Inc., Collegeville, PA 19426 (US)
(72) Inventor: PETERMANN, Oliver, 29699 Bomlitz (DE); KNARR, Matthias, 29699 Bomlitz (DE)
(74) Representative: f & e patent
(86) International application number: PCT/US2019/066724
(87) International publication number: WO 2020/131792

(56) References cited:
- CN-A- 1 891 218
- SUDHIR KARNA ET AL: "SWELLABLE AND FLOATING GASTRORETENTIVE FORMULATION FOR SUSTAINED DELIVERY OF METFORMIN HCL", INTERNATIONAL JOURNAL OF PHARMACEUTICAL SCIENCES AND RESEARCH IJPSR, 1 January 2016 (2016-01-01), pages 1590 - 1602, XP055590479, Retrieved from the Internet <URL:http://ijpsr.com/bft-article/swellable-and-floating-gastroretentive-formulation-for-sustained-delivery-of-metformin-hcl/?view=fulltext>
- UNKNOWN: "Ethylcellulose", ORAL CONTROLLED RELEASE FORMULATION DESIGN AND DRUG DELIVERY : THEORY TO PRACTICE, 1 January 2011 (2011-01-01), ProQuest Ebook Central, pages 80, XP055590065, Retrieved from the Internet <URL:https://ebookcentral.proquest.com/lib/epo-ebooks/detail.action?docID=588947&query=oral+controlled+release+formulation+design+and+drug> [retrieved on 20190520]
- T WUESTENBERG: "Ethylcellulose", CELLULOSE AND CELLULOSE DERIVATIVES IN THE FOOD INDUSTRY: FUNDAMENTALS AND APPLICATIONS, FIRST EDITION, 1 January 2014 (2014-01-01), ProQuest Ebook Central, pages 275 - 342, XP055590053, Retrieved from the Internet <URL:https://ebookcentral.proquest.com/lib/epo-ebooks/reader.action?docID=1762799&query=cellulose%2Band%2Bcellulose%2Bderivatives%2Bin%2Bthe%2Bfood%2Bindustry> [retrieved on 20190520]

## Description

### FIELD

The present invention relates to novel sustained release compositions comprising a physiologically active ingredient and an ethylcellulose.

### INTRODUCTION

Sustained release dosage forms have found wide application in a variety of technology areas such as in personal care and agricultural applications, water treatment and in particular pharmaceutical applications. Sustained release dosage forms are designed to release a finite quantity of an active ingredient into an aqueous environment over an extended period of time. Sustained release pharmaceutical dosage forms are desirable because they provide a method of delivering a long-lasting dose in a single application without overdosing. Known sustained release pharmaceutical dosage forms contain a drug or a vitamin whose release is controlled by a polymeric matrix which, for instance, may comprise one or more water-soluble cellulose ethers. Water-soluble cellulose ethers hydrate on the surface of a tablet to form a gel layer. A fast formation of the gel layer is important to prevent wetting of the interior and disintegration of the tablet core. Once the gel layer is formed, it controls the penetration of additional water into the tablet. As the outer layer fully hydrates and dissolves, an inner layer must replace it and be sufficiently cohesive and continuous to retard the influx of water and control drug diffusion.

A commonly used cellulose ether for providing sustained release of an active ingredient from an oral dosage form is hydroxypropyl methylcellulose (HPMC). For instance, US 4,734,285 discloses that the release of an active ingredient can be prolonged by employing a fine particle sized HPMC as an excipient in a solid tablet. HPMC is used in commercial oral pharmaceutical formulations as a component of a polymeric matrix providing sustained release of a drug usually at a concentration of 30% to 60% by weight of the oral dosage form.

EP 1978940 B1 relates to a composition for controlled release of an active ingredient, the composition comprising at least 25% by weight of ethylcellulose and at least 10% by weight of polyethylene oxide. The composition is prepared by hot melt extrusion. Karna et al. IJPSR, 2016; Vol. 7(4): 1590-1602 disclose swellable sustained release tablets comprising metformin and ethyl cellulose. Patent document CN 1891218 discloses sustained release formulations comprising ranolazine and ethyl cellulose.

It is a well-known problem in the pharmaceutical art that some patients, especially children or the elderly, or patients with dysphagia, find it difficult to swallow conventional oral dosage forms such as capsules or tablets. In particular, this is the case if the drug administered in the dosage form is a highly dosed drug which, when the drug is formulated with pharmaceutical excipients in the typical amounts included in commercial dosage forms, either makes each dosage form very large or requires the dose to be divided among two or more dosage forms that have to be swallowed at the same time.

It would therefore be desirable to develop a sustained release oral dosage form where a drug is formulated with a reduced amount of excipient(s) to permit a reduction in the overall size of the dosage form and improve the swallowability without compromising the sustained release properties thereof.

### SUMMARY OF THE INVENTION

It has surprisingly been found that when a water-insoluble ethylcellulose is used as an excipient in a mixture with a physiologically active ingredient, it is capable of forming a stable hydrogel at a temperature of 37°C and provide sustained release of the active ingredient. This is the case even when it is used at much lower concentrations that the concentrations of HPMC used in commercial formulations and at much lower concentrations than in the melt-extruded compositions disclosed in EP 1978940 B1.

Accordingly, the present invention relates to a sustained release solid composition for oral administration comprising a physiologically active ingredient embedded in a polymeric matrix of a water-insoluble ethylcellulose which has a DS(ethyl) of at least 1.8, wherein the concentration of ethylcellulose is 1.5-10% by dry weight of the active ingredient, and wherein the ethylcellulose constitutes 80-100% by weight of the polymeric matrix.

### BRIEF DESCRIPTION OF THE DRAWINGS

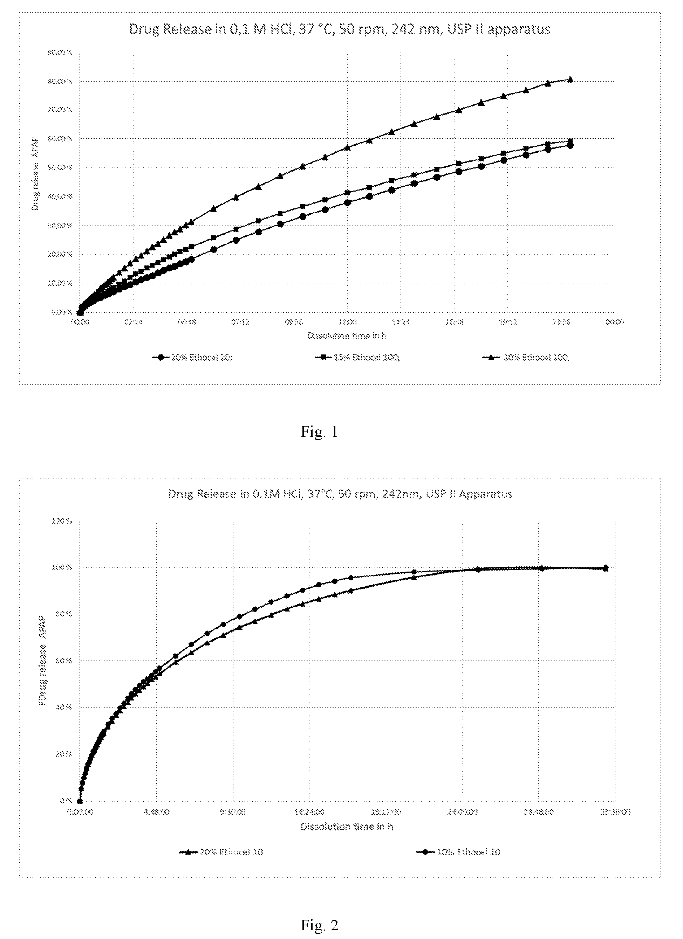

Fig. 1 is a graph showing the release over time of acetaminophen (APAP) from compositions of the invention containing solutions of ETHOCEL Standard 20 Premium (20% solution, shown as - • -) and ETHOCEL Standard 100 Premium (10% and 15% solutions, shown as - ▲ - and - ■ -, respectively) when HPMC capsules containing the dried compositions were immersed in 900 ml of 0.1 N HCl, pH 1.1.
Fig. 2 is a graph showing the release over time of APAP from compositions of the invention containing a 10% by weight and 20% by weight solutions of ETHOCEL Std. 10 FP (shown as - • - and - ▲ -, respectively) when compressed tablets containing the dried composition was immersed in 900 ml of 0.1 N HCl, pH 1.1.

### DESCRIPTION OF EMBODIMENTS

In the present invention, ethylcellulose is an essential component of the composition to form a hydrogel in an aqueous environment such as the stomach and provide sustained release of the active ingredient on oral administration of the composition even when the ethylcellulose is present in a very low concentration relative to the active ingredient.

The ethylcellulose is composed of anhydroglucose units joined by 1-4 linkages. Each anhydroglucose unit contains hydroxyl groups at the 2, 3 and 6 positions. Partial or complete substitution of these hydroxyls creates cellulose derivatives. For example, treatment of cellulosic fibers with caustic solution, followed by an ethylating agent, yields cellulose ethers substituted with one or more ethoxy groups. If not further substituted with other alkyls, this cellulose derivative is known as ethylcellulose.

The present inventors have surprisingly found that ethylcellulose wherein hydroxy groups of the anhydroglucose units are substituted with ethyl groups to a DS (ethyl) of more than 1.8 can form a stable hydrogel at about 37°C when included in the composition at concentrations that are sufficient to embed particles of the active ingredient. While such concentrations may vary between wide limits, and while generally more sustained release may be obtained at higher concentrations of ethylcellulose (e.g. 30-60% by weight), it has surprisingly been found that ethylcellulose at low concentrations, i.e. concentrations of 1.5-10% by dry weight of the active ingredient, may be sufficient to cause the active ingredient to become embedded to an extent providing sustained release of the active ingredient over 24 hours. The concentration of ethylcellulose included in the present composition is 1.5-10% and most preferably 2.5-5% by dry weight of the active ingredient. Compositions containing about 4.0-4.5% of ethylcellulose by dry weight of the active ingredient have been shown to provide a release of the active ingredient of between about 60% and 95% over a period of 22 hours, cf. Examples 1-5 below. The resulting sustained release dosage form, such as tablet or capsule, is smaller in size and therefore easier to ingest. It has furthermore been found that a satisfactory release rate may be obtained without adding any other excipients to the dosage form, though a surfactant may optionally be added during the manufacturing process as a defoaming agent.

The ethylcellulose preferably has a DS(ethyl) of from 2.0 to 3.0, more preferably from 2.2 to 2.8, even more preferably from 2.3 to 2.7, still more preferably from 2.4 to 2.65 and most preferably from 2.5 to 2.6. The degree of the ethyl substitution, DS(ethyl), also designated as DS(ethoxyl), of an ethylcellulose is the average number of OH groups substituted with ethyl groups per anhydroglucose unit.

The determination of the % ethoxyl in ethylcellulose is carried out by a Zeisel gas chromatographic technique as described in ASTM D4794-94(2003). These are subsequently converted into degree of substitution (DS) for ethyl substituents according to the formulas below: % cellulose backbone = 100-[%EtO*[[M(OCH2-CH3)-M(OH)]/M(OCH2-CH3)] DS(ethyl) = [[%EtO/M(OCH2-CH3)]/(%cellulose backbone/M(AGU))] wherein EtO is ethoxy and AGU is anhydroglycose unit

Residual amounts of salt have been taken into account in the conversion.

The viscosity of the ethylcellulose is generally from 3 to 110 mPa.s when measured as a 5 wt. % solution in a mixture of toluene and ethanol in a weight ratio of 80:20 at 25°C in an Ubbelohde viscometer. The viscosity of the ethylcellulose may be from 15 to 30 mPa.s and more preferably from 18 to 25 mPa.s when measured as indicated above. Alternatively, the viscosity of the ethylcellulose may be from 90 to 110 mPa.s when measured as indicated above.

In an aqueous environment, the ethylcellulose is capable of gelling at 37°C at very low concentrations, forming stable hydrogels in an aqueous environment. The term "stable hydrogels", when used in this context is intended to mean hydrogels that retain their shape and are not completely dissolved or significantly eroded after immersion in 0.1 N HCl, pH 1.1, for 4 hours at 37°C.

Examples of ethylcelluloses are ETHOCEL Standard 20 Premium ethylcellulose, ETHOCEL Standard 100 Premium ethylcellulose and ETHOCEL Standard 10 FP ( available from DuPont).

The ethylcellulose is useful as an excipient for a sustained release dosage form which means that it has the function to regulate the release of an active ingredient from the dosage form over an extended period of time. The term "sustained release" is used herein synonymously with the term "controlled release". Sustained release is an approach by which active ingredients such as physiologically active compounds are made available at a rate and duration designed to accomplish an intended effect. The ethylcellulose is useful for forming all or part of a polymeric matrix in which the active ingredient is embedded. The polymeric matrix may additionally comprise one or more other polymers capable of providing sustained release of the active ingredient from the dosage form. The ethylcellulose constitutes 80-100%, and most preferably 90-100% by weight of the polymeric matrix. The polymeric matrix may include one or more other polymers such as other celluloseethers, e.g. hydroxypropyl methylcellulose, hydroxyethyl methylcellulose, methylcellulose, hydroxypropyl cellulose or carboxymethyl cellulose, or it may include one or more other polysaccharides such as sodium alginate or calcium alginate. It is generally preferred that ethylcellulose constitutes 100% by weight of the polymeric matrix.

The ethylcellulose may be included in sustained release dosage forms, in particular dosage forms intended for oral administration of drugs or other physiologically active ingredients and release thereof into the gastrointestinal tract so as to control the absorption rate of the active ingredient to achieve a desired blood plasma profile. The combined amount of ethylcellulose and active ingredient in the dosage form is preferably at least 50%, more preferably 70% and most preferably at least 95% by dry weight of the dosage form, and preferably up to 100%, more preferably up to 98% and most preferably up to 95% by dry weight of the dosage form. The dosage form is designed to provide a constant or nearly constant level of the active ingredient in plasma with reduced fluctuation via a slow, continuous release of the active ingredient over an extended period of time such as a period of between 4 and 30 hours, preferably between 8 and 24 hours to release all or almost all of the active ingredient from the dosage form.

It has been found that sustained release dosage forms such as tablets and capsules wherein the polymer matrix is formed partially or completely from ethylcellulose remains intact over an extended time period such as at least 4 hours, preferably at least 6 hours and under optimized conditions at least 8 hours. Without wanting to be bound by theory, it is believed that the ethylcellulose is hydrated to form a strong swollen layer on the outer surface of the dosage form upon contact with an aqueous liquid at body temperature. The strong swollen layer minimizes the release of the active ingredient caused by erosion of the dosage form. Since the tablets or capsule contents do not disintegrate (i.e. do not fall apart to any significant degree), the release of the active ingredient is controlled by the slow diffusion from the swollen layer that has been formed by hydration of the ethylcellulose on the outer surface of the dosage form. A strong swollen layer reduces the penetration of water into the sustained release dosage form, which delays the release of the active ingredient, particularly a water-soluble active ingredient, into an aqueous environment due to a reduced amount of water in the zone of the dosage form into which water diffuses and dissolves the active ingredient. It is considered most surprising that ethylcellulose, which is not soluble in water, is capable of being hydrated and forming a hydrogel in this manner.

In an embodiment, the composition comprises an additive which on ingestion reacts with gastric fluid to generate a gas such as CO₂. The developing gas is trapped in the hydrogel which, as a result, floats to the surface of the gastric contents resulting in prolonged gastric retention time. The prolonged gastric retention time improves the bioavailability of the active ingredient, increases the duration of release and improves the solubility of active ingredients that are not readily soluble in the high pH environment of the intestine. Examples of additives which generate gas in contact with gastric fluid are alkali metal or alkaline earth metal carbonates such as CaCO₃ or Na₂CO₃ . The concentration of the additive may be in the range of 1-5% by weight, preferably 1.5-3% by weight, such as 2% by weight of the composition.

Addition of a surfactant helps to distribute a low level of liquid diluent homogenously and produce a smooth highly viscous semi-solid paste, possibly due to defoaming and emulsification. The surfactant may be selected from conventional defoaming agents selected from the group consisting of anionic surfactants with anionic functional groups such as sulfates, sulfonates, phosphates and carboxylates such as alkyl sulfates, e.g. ammonium lauryl sulfate, sodium lauryl sulfate (sodium dodecyl sulfate, SLS, or SDS), and alkyl-ether sulfates, such as sodium laureth sulfate (sodium lauryl ether sulfate or SLES), and sodium myreth sulfate; cationic surfactants with cationic functional groups such as cetrimonium bromide (CTAB), cetylpyridinium chloride (CPC), benzalkonium chloride (BAC), benzethonium chloride (BZT), dimethyldioctadecylammonium chloride, dioctadecyldimethylammonium bromide (DODAB); zwitterionic surfactants such as cocamidopropyl betaine; and nonionic surfactants such as siloxane surfactants like modified polydimethylsiloxane-based defoamer, ethoxylates, fatty acid esters of glycerol, sorbitol and sucrose. The concentration of surfactant may be in the range of 0.1-1.5% by weight of the composition.

In one embodiment of the invention, the composition comprising ethylcellulose admixed with the active ingredient is in the form of a dry powder. The dry powder may be prepared by preparing a solution of ethylcellulose in an organic solvent such as ethanol, methanol, isopropanol or acetone or a mixture thereof with water, and mixing the solution with an active ingredient and optionally one or more solid excipients, and drying the mixture at a temperature of 40-100°C until the mixture has a moisture content of less than 10% by weight, preferably less than 5% by weight, more preferably less than 3% by weight, in particular less than 2% by weight, such as less than 1% by weight, followed by milling or grinding the mixture to granules of a desired particle size in a manner known in the art. The dry powder will typically contain granules comprising the active ingredient partially or completely encased by ethylcellulose which facilitates sustained release of the active ingredient as discussed above.

In one embodiment, the invention relates to a unit dosage form comprising the present composition. The unit dosage form is intended for oral administration and may be in the form of a tablet comprising the compressed dried composition, for instance in the form of compressed granules of the dried composition. Alternatively, the unit dosage form may be in the form of a tablet or pellet prepared by extruding the semi-solid paste prepared as described above and cutting the extruded mass into pieces of an appropriate size followed by drying. The tablet may optionally comprise one or more other excipients, such as a cellulose derivative as described above, though preferably ethylcellulose is the only polymeric matric forming excipient included in the dosage form, except that a surfactant may optionally also be included as indicated above. The unit dosage form may also be a capsule including the dried composition, preferably in the form of dry granules containing the mixture of methylcellulose and active ingredient. The unit dosage form may also be in the form of a syringe or pouch pre-filled with the wet mixture: this dosage form may more readily be administered to young children.

The unit dosage form contains one or more physiologically active ingredients, preferably one or more drugs, one or more diagnostic agents, or one or more physiologically active ingredients which are useful for cosmetic or nutritional purposes. The term "drug" denotes a compound having beneficial prophylactic and/or therapeutic properties when administered to an individual, typically a mammal, especially a human individual. The dosage form is believed to be particularly suited for administering highly dosed drugs, i.e. drugs administered in unit doses of 500 mg or more, as it is possible to provide a unit dose that includes the requisite amount of the active ingredient in a size that makes it easier to ingest. Examples of highly dosed drugs are metformin, metformin hydrochloride, acetaminophen (paracetamol) or acetylsalicylic acid. Thus, each unit dosage form may typically include 500-1000 mg of the active ingredient.

Some embodiments of the invention will now be described in detail in the following Examples.

Unless otherwise mentioned, all parts and percentages are by weight. In the Examples the following test procedures are used.

### Ubbelohde measurement (ethylcellulose)

In typical embodiments, the ethylcellulose has a viscosity of from 3 to 110 mPa.s in a 5% solution in a mixture of toluene and ethanol at a weight ratio of 80:20. The viscosity is determined at 25°C in an Ubbelohde viscometer. A typical viscosity analysis is performed as follows: 57 g of a 80:20 toluene/ethanol mixture is weighed into a dry 8 ounce (ca. 240 ml) bottle and 3 g of ethylcellulose is added. The bottle is placed on a mechanical shaker and shaken until all the ethylcellulose is dissolved (approximately for 20 minutes). The resulting solution is analyzed within 24 h of preparation. For viscosity measurement, the solution is filled into an Ubbelohde viscometer which is then placed in a water bath at 25°C until the solution has equilibrated to 25°C. Following the instructions for the Ubbelohde viscometer, the solution is sucked up through the calibration flow tube and then allowed to drain. The time of the flow between the upper and lower calibration mark is stopped and the viscosity is calculated according to the instructions taking into account the specific capillary used for the measurement.

### Example 1: Release of Acetaminophen from dried gelatin capsules comprising ethylcellulose

A 20 % by weight solution of ethylcellulose (ETHOCEL Std. 20 Premium, available from DuPont) in ethanol was prepared. 7.00 g of finely ground acetaminophen (abbreviated herein to APAP) was intimately mixed with 3.00 g of the ethylcellulose solution until a white homogenous and highly viscous paste was obtained. The mixture was immediately filled into a syringe and injected into HPMC capsules (size 00) which were subsequently closed and sealed. The mixture was carefully dried overnight at 60°C.

The dried capsules were placed in 900 ml of 0.1N HCl pH 1.1 at 37°C and drug release was measured in a USP II dissolution apparatus at 37°C, 100 rpm, for 22 hours at a wavelength of 243 nm with a path length of 0.1 mm.

The release of APAP from the dried capsules is shown in Fig. 1 from which it appears that about 60% of the drug was released after 22 hours (shown as **-•** - in the figure).

### Example 2: Release of Acetaminophen from dried gelatin capsules comprising ethylcellulose

A 10% by weight solution of ethylcellulose (ETHOCEL Std. 100 Premium, available from DuPont) in ethanol was prepared. 7.00 g of finely ground acetaminophen (abbreviated herein to APAP) was intimately mixed with 3.00 g of the ethylcellulose solution until a white homogenous and highly viscous paste was obtained. The mixture was immediately filled into a syringe and injected into HPMC capsules (size 00) which were subsequently closed and sealed. The mixture was carefully dried overnight at 60°C.

The dried capsules were placed in 900 ml of 0.1N HCl pH 1.1 at 37°C and drug release was measured in a USP II dissolution apparatus at 37°C, 100 rpm, for 22 hours at a wavelength of 243 nm with a path length of 0.1 mm.

The release of APAP from the dried capsules is shown in Fig. 1 from which it appears that about 60% of the drug was released after 22 hours (shown as **-▲-** in the figure).

### Example 3: Release of Acetaminophen from dried gelatin capsules comprising ethylcellulose

A 15% by weight solution of ethylcellulose (ETHOCEL Std. 100 Premium, available from DuPont) in ethanol was prepared. 7.00 g of finely ground acetaminophen (abbreviated herein to APAP) was intimately mixed with 3.00 g of the ethylcellulose solution until a white homogenous and highly viscous paste was obtained. The mixture was immediately filled into a syringe and injected into HPMC capsules (size 00) which were subsequently closed and sealed. The mixture was carefully dried overnight at 60°C.

The dried capsules were placed in 900 ml of 0.1N HCl pH 1.1 at 37°C and drug release was measured in a USP II dissolution apparatus at 37°C, 100 rpm, for 22 hours at a wavelength of 243 nm with a path length of 0.1 mm.

The release of APAP from the dried capsules is shown in Fig. 1 from which it appears that about 80% of the drug was released after 22 hours (shown as **-■** - in the figure).

### Example 4: Release of acetaminophen from compressed tablets containing ethylcellulose

A 10% by weight solution of ethylcellulose (ETHOCEL Std. 10 FP, available from DuPont) in ethanol was prepared. 7.00 g of finely ground acetaminophen (abbreviated herein to APAP) was intimately mixed with 3.00 g of the ethylcellulose solution until a white homogenous and viscous paste was obtained. The mixture was spread out onto a plate and dried at 60°C. When completely dry, the mixture was de-agglomerated by hand using a pistil and compressed into tablets by using a lab manual IR tablet press at a pressure of 2t. The tablets had a weight of 500 mg and contained 95.89% by weight of APAP. The tablets had a tablet hardness of 32 N.

The tablets were placed in 900 ml of 0.1N HCl pH 1.1 at 37°C and drug release was measured in a USP II dissolution apparatus at 37°C, 100 rpm, for 22 hours at a wavelength of 243 nm with a path length of 0.1 mm.

The release of APAP from the tablets is shown in Fig. 2 from which it appears that about 95% of the drug was released after 22 hours (shown as **-•** - in the figure).

### Example 5: Release of acetaminophen from compressed tablets containing ethylcellulose

A 20% by weight solution of ethylcellulose (ETHOCEL Std. 10 FP, available from DuPont) in ethanol was prepared. 7.00 g of finely ground acetaminophen (abbreviated herein to APAP) was intimately mixed with 3.00 g of the ethylcellulose solution until a white homogenous and viscous paste was obtained. The mixture was spread out onto a plate and dried at 60°C. When completely dry, the mixture was de-agglomerated by hand using a pistil and compressed into tablets by using a lab manual IR tablet press at a pressure of 2t. The tablets had a weight of 505 mg and contained 92.11% by weight of APAP. The tablets had a tablet hardness of 42 N.

The tablets were placed in 900 ml of 0.1N HCl pH 1.1 at 37°C and drug release was measured in a USP II dissolution apparatus at 37°C, 100 rpm, for 22 hours at a wavelength of 243 nm with a path length of 0.1 mm.

The release of APAP from the tablets is shown in Fig. 2 from which it appears that about 95% of the drug was released after 22 hours (shown as **-▲-** in the figure).

## Claims

1. A sustained release solid composition for oral administration comprising a physiologically active ingredient embedded in a polymeric matrix of a water-insoluble ethylcellulose which has a DS(ethyl) of at least 1.8, wherein the concentration of ethylcellulose is 1.5-10% by dry weight of the active ingredient, and wherein the ethylcellulose constitutes 80-100% by weight of the polymeric matrix.

2. The composition of claim 1, wherein the concentration of ethylcellulose is 2.5-5% by dry weight of the active ingredient.

3. The composition of claim 1 or 2, wherein the ethylcellulose has a DS(ethyl) of from 2.0 to 3.0, preferably from 2.2 to 2.8, more preferably from 2.3 to 2.7, still more preferably from 2.4 to 2.65 and most preferably from 2.5 to 2.6.

4. The composition of any one of claims 1 to 3, wherein the ethylcellulose has a viscosity of from 3 to 110 mPa•s, measured as 5 % by weight solution in a mixture of toluene and ethanol in a weight ratio of 80:20 at 25 °C in an Ubbelohde viscometer.

5. The composition of any one of claims 1 to 4, wherein the ethylcellulose constitutes 90-100% by weight of the polymeric matrix.

6. The composition of any of claims 1 to 5 further comprising a surfactant.

7. The composition of any one of claims 1 to 6, wherein the concentration of the surfactant is in the range of 0.1-1.5% by weight of the composition.

8. The composition according to any one of claims 1 to 7 further comprising an additive capable of reacting with gastric fluid to generate a gas.

9. The composition according to claim 8, wherein the additive is selected from alkali metal or alkaline earth metal carbonates such as CaCO₃ or Na₂CO₃.

10. The composition of any one of claims 1 to 9 in the form of a dry powder.

11. The composition of any one of claims 1 to 10, wherein the active ingredient is selected from the group consisting of metformin, metformin hydrochloride, acetaminophen and acetylsalicylic acid.

12. A unit dosage form comprising a composition according to any one of claims 1 to 11.

13. The unit dosage form of claim 12 which is a tablet comprising a compressed dried composition according to any one of claims 1 to 11.

14. The unit dosage form of claim 13, wherein the tablet comprises compressed granules of a dried composition according to any one of claims 1 to 11.

## Patentansprüche

1. Feste Zusammensetzung mit verzögerter Freisetzung zur oralen Verabreichung, enthaltend einen physiologisch aktiven Inhaltsstoff, der in eine polymere Matrix aus einer wasserunlöslichen Ethylcellulose eingebettet ist, die einen DS(ethyl) von mindestens 1,8 aufweist, wobei die Konzentration der Ethylcellulose 1,5-10 % des Trockengewichts des aktiven Inhaltsstoffs beträgt, und wobei die Ethylcellulose 80-100 % des Gewichts der polymeren Matrix ausmacht.

2. Zusammensetzung gemäß Anspruch 1, wobei die Konzentration der Ethylcellulose 2,5-5 % des Trockengewichts des aktiven Inhaltsstoffs beträgt.

3. Zusammensetzung gemäß Anspruch 1 oder 2, wobei die Ethylcellulose einen DS(ethyl) von 2,0 bis 3,0, vorzugsweise von 2,2 bis 2,8, noch bevorzugter von 2,3 bis 2,7, noch weiter bevorzugt von 2,4 bis 2,65 und am meisten bevorzugt von 2,5 bis 2,6 aufweist.

4. Zusammensetzung gemäß einem der Ansprüche 1 bis 3, wobei die Ethylcellulose eine Viskosität von 3 bis 110 mPa·s aufweist, gemessen als 5 Gew.% Lösung in einem Gemisch aus Toluol und Ethanol in einem Gewichtsverhältnis von 80:20 bei 25 °C in einem Ubbelohde-Viskosimeter.

5. Zusammensetzung gemäß einem der Ansprüche 1 bis 4, wobei die Ethylcellulose 90-100 Gew.-% der Polymermatrix ausmacht.

6. Zusammensetzung gemäß einem der Ansprüche 1 bis 5, außerdem enthaltend ein Tensid.

7. Zusammensetzung gemäß einem der Ansprüche 1 bis 6, wobei die Konzentration des Tensids im Bereich von 0,1-1,5 Gew.% der Zusammensetzung liegt.

8. Zusammensetzung gemäß einem der Ansprüche 1 bis 7, außerdem enthaltend ein Additiv, das in der Lage ist, mit Magenflüssigkeit zu reagieren, um ein Gas zu erzeugen.

9. Zusammensetzung gemäß Anspruch 8, wobei das Additiv aus Alkali- oder Erdalkalimetallcarbonaten wie CaCO₃ oder Na₂CO₃ ausgewählt ist.

10. Zusammensetzung gemäß einem der Ansprüche 1 bis 9 in Form eines trockenen Pulvers.

11. Zusammensetzung gemäß einem der Ansprüche 1 bis 10, wobei der aktive Inhaltsstoff ausgewählt ist aus der Gruppe bestehend aus Metformin, Metforminhydrochlorid, Acetaminophen und Acetylsalicylsäure.

12. Einheitsdosierungsform, enthaltend eine Zusammensetzung gemäß einem der Ansprüche 1 bis 11.

13. Einheitsdosierungsform gemäß Anspruch 12, die eine Tablette darstellt, die eine verpresste, getrocknete Zusammensetzung gemäß einem der Ansprüche 1 bis 11 umfasst.

14. Einheitsdosierungsform gemäß Anspruch 13, wobei die Tablette ein verpresstes Granulat einer getrockneten Zusammensetzung gemäß einem der Ansprüche 1 bis 11 umfasst.

## Revendications

1. Composition solide à libération retardée pour administration orale comprenant un composant physiologiquement actif incorporé dans une matrice polymère comprenant une éthylcellulose insoluble dans l'eau, qui présente un DS(éthyle) d'au moins 1,8, dans laquelle la concentration d'éthylcellulose est 1,5-10 % en poids sec du composant actif, et dans laquelle l'éthylcellulose constitue 80-100 % en poids de la matrice polymère.

2. Composition selon la revendication 1, dans laquelle la concentration d'éthylcellulose est 2,5-5 % en poids sec du composant actif.

3. Composition selon la revendication 1 ou 2, dans laquelle l'éthylcellulose présente un DS(éthyle) de 2,0 à 3,0, de préférence de 2,2 à 2,8, plus préférablement de 2,3 à 2,7, encore plus préférablement de 2,4 à 2,65 et de façon tout particulièrement préférée de 2,5 à 2,6.

4. Composition selon l'une quelconque des revendications 1 à 3, dans laquelle l'éthylcellulose a une viscosité de 3 à 110 mPa·s, mesurée sous forme d'une solution à 5 % en poids dans un mélange de toluène et d'éthanol en un rapport en poids de 80:20 à 25 °C dans un viscosimètre Ubbelhode.

5. Composition selon l'une quelconque des revendications 1 à 4, dans laquelle l'éthylcellulose constitue 90-100 % en poids de la matrice polymère.

6. Composition selon l'une quelconque des revendications 1 à 5, comprenant en outre un tensioactif.

7. Composition selon l'une quelconque des revendications 1 à 6, dans laquelle la concentration du tensioactif se situe dans la plage de 0,1-1,5 % en poids de la composition.

8. Composition selon l'une quelconque des revendications 1 à 7, comprenant en outre un additif pouvant réagir avec le liquide gastrique pour produire un gaz.

9. Composition selon la revendication 8, dans laquelle l'additif est choisi parmi les carbonates de métaux alcalins ou de métaux alcalino-terreux tels que CaCO₃ ou Na₂CO₃.

10. Composition selon l'une quelconque des revendications 1 à 9, sous la forme d'une poudre sèche.

11. Composition selon l'une quelconque des revendications 1 à 10, dans laquelle le composant actif est choisi dans l'ensemble constitué par la metformine, le chlorhydrate de metformine, l'acétaminophène et l'acide acétylsalicylique.

12. Forme pharmaceutique unitaire comprenant une composition selon l'une quelconque 1 à 11.

13. Forme pharmaceutique unitaire selon la revendication 12, qui est un comprimé comprenant une composition séchée comprimée selon l'une quelconque des revendications 1 à 11.

14. Forme pharmaceutique unitaire selon la revendication 13, dans laquelle le comprimé comprend des granules comprimés d'une composition séchée selon l'une quelconque des revendications 1 à 11.
